# EUROPÄISCHE PATENTANMELDUNG

(11) **EP 4 194 422 A1**
(43) Veröffentlichungstag der Anmeldung: **14.06.2023**
(21) Anmeldenummer: 22208750.4
(22) Anmeldetag: 22.11.2022
(51) Int. Cl.: C04B 26/00, C04B 18/24, C08K 11/00, C08K 9/04, C09C 3/08, B29C 39/02, B29C 39/00

(54) **VERFAHREN ZUR HERSTELLUNG VON HYDROPHOBIERTEN UND REAKTIVEN ANORGANISCHEN UND/ODER ORGANISCHEN FÜLLSTOFFEN, DERART HERGESTELLTE FÜLLSTOFFE SOWIE AUS EINER POLYMERBASIERTEN GIESSMASSE ENTHALTEND WENIGSTENS EINEN SOLCHEN FÜLLSTOFF HERGESTELLTES FORMTEIL**

(30) Priorität: 09.12.2021 DE 102021132486
(71) Anmelder: Schock GmbH, 94209 Regen (DE)
(72) Erfinder: Datsyuk, Vitaliy, 94227 Zwiesel (DE); Orendorz, Adam, 94227 Zwiesel (DE)
(74) Vertreter: Lindner Blaumeier Patent- und Rechtsanwälte

(57) **Zusammenfassung**

Verfahren zur Herstellung von hydrophoben und reaktiven anorganischen und/oder organischen Füllstoffen, umfassend die Schritte: (a) Bereitstellen eines Füllstoffs mit einer bestimmten Oberfläche, (b) Mischen des Füllstoffs mit der Lösung von mindestens einer hydrophobierenden und aktivierenden reaktiven Verbindung auf biologischer Basis in einem Mischaggregat in einer Menge von 0,15 × 10⁻² bis 5,0 × 10⁻² g pro m² Füllstoffoberfläche bei einer Geschwindigkeit von 20 U/min bis 200 U/min für 12 Minuten bis 120 Minuten, (c) Evakuieren des hydrophobierten und aktivierten anorganischen und/oder organischen Füllstoffs in einem Lagerbeutel, einer Box oder einem Fass oder direkt in der Gießmasse.

## Beschreibung

Die Erfindung betrifft ein Verfahren zur Herstellung von hydrophobierten und reaktiven anorganischen und/oder organischen Füllstoffen. Solche Füllstoffe werden z.B. als Zuschlag zu polymerbasierten Gießmassen, aus denen Komposit-Formkörper hergestellt werden, verwendet.

Schrumpfung und Schrumpfungsstress sind ein häufiges Problem bei vielen Anwendungen, die auf radikalisch gehärteten duroplastischen Materialien basieren, wie z. B. geformte Küchenspülen, Waschbecken, Badewannen oder Zahnfüllungs-Verbundsysteme. Solche Verbundwerkstoffe enthalten in der Regel ein radikalisch gehärtetes Polymerbindemittel, ein Initiatoren-System und mit Silan behandelte anorganische Füllstoffpartikel. Bei der Aushärtung dieser Komposit-Systeme wird eine Schrumpfung beobachtet, die einerseits Mikrorisse und andererseits starke intrinsische Spannungen im Material hervorrufen kann. Bei geformten Küchenspülen beispielsweise kann dies zu einer Rissausbreitung im Material führen, die ein Austreten von Wasser oder eine Verringerung der mechanischen Eigenschaften zur Folge hat. Die gleichen Probleme können bei zahnmedizinischen Kompositmaterialien auftreten: Spannungen, Mikroleckagen, Ablösung des Klebers und schließlich Schmerzen für den Patienten.

Dieses Problem kann auf den hohen Füllstoffgehalt in Kompositmaterialien und die Verwendung von 3-Methacryloxypropyltrimethoxysilan zurückgeführt werden, das auf der Oberfläche der Quarzpartikel immobilisiert ist und mit seiner relativ kurzen Dreifachkette eine geringere Mobilität aufweist. Während der Polymerisation führt diese eingeschränkte Mobilität zu einem schnellen Abbruch der Radikale und zur Bildung kurzer Polymerketten, was aufgrund der hohen Steifigkeit zu einer starken Belastung des Systems führt. Thermisch bedingte Kontraktionen oder Expansionen des Materials sowie mechanische Stöße können als Quelle für Mikrorisse an der Füllstoff-Matrix-Grenzfläche wirken.

An der Füllstoff-Matrix-Grenzfläche konzentrieren sich die natürlichen Spannungen aufgrund des Vorhandenseins vieler reaktiver Gruppen an einer starren, anorganischen Oberfläche. Die Oberfläche der Quarzsandpartikel weist in der Regel 0,8 Hydroxylgruppen auf 1 Quadratnanometer der Oberfläche auf. Während des Silanisierungsprozesses reagiert fast jede Hydroxylgruppe mit einem einzelnen Silanmolekül und bildet eine einheitliche hydrophobe Silanschicht, was zu einem superhydrophoben Effekt mit einer sehr dichten Immobilisierung der reaktiven Methacrylatgruppe führt. Die hohe Konzentration der Doppelbindungen, die auf der Füllstoffoberfläche immobilisiert sind, resultiert in der Polymerisation der kurzen Polymerketten und damit in der Entstehung von Bereichen mit einer hohen Eigenspannung. Die Spannungen können durch den Ersatz des Methacrylolsilans durch nicht reaktive Silane verringert werden, was jedoch die mechanischen Eigenschaften wie z.B. die Schlagzähigkeit aufgrund der fehlenden Bindung zwischen Füllstoff und Matrix verringert.

Die Verwendung der mit Silan behandelten, anorganischen und/oder organischen Füllstoffe, die mit einem Silan-Kupplungsmittel behandelt wurden, erfordert die Hydrolyse der hydrolysierbaren Esterfunktionen des Silans. Die Hydrolyse von beispielsweise 1000 kg 3-Methacryloxypropyltrimethoxysilan führt zur Herstellung von 387 kg Methanol, einer brennbaren Flüssigkeit mit hohem Dampfdruck, die beim Verschlucken tödlich sein kann.

Weiterhin erfolgt die Silanisierung von Füllstoffen technisch mittels einer thermischen Aktivierung bei Temperaturen von 60°C oder höher. Die für diesen Prozess notwendige Energie wird in der Regel über das Verbrennen von Erdgas bzw. entsprechende Kohlenwasserstoffe realisiert, die zu einem CO₂-Ausstoß beitragen und die Prozesskosten erhöhen. Nach dem Silanisierungsprozess ist in der Regel eine Reifezeit von mehreren Tagen notwendig, bis sich die gewünschte Hydrophobizität des Füllstoffes einstellt.

Aufgabe der vorliegenden Erfindung ist es, die oben beschriebenen, technischen und umweltrelevanten Probleme des Standes der Technik zu beseitigen.

Zur Lösung der Aufgabe wird ein Verfahren zur Herstellung von hydrophoben und reaktiven anorganischen und/oder organischen Füllstoffen vorgeschlagen, umfassend die Schritte: (a) Bereitstellen eines Füllstoffs mit einer bestimmten Oberfläche, (b) Mischen des Füllstoffs mit der Lösung von mindestens einer hydrophobierenden und aktivierenden reaktiven Substanz auf biologischer Basis in einem Mischaggregat in einer Menge von 0,15 × 10⁻² bis 5,0 × 10⁻² g/m² pro m² Füllstoffoberfläche bei einer Geschwindigkeit von 20 U/min bis 200 U/min für 12 Minuten bis 120 Minuten, (c) Umlagern des hydrophobierten und aktivierten anorganischen und/oder organischen Füllstoffs in einen Lagerbeutel, einer Box oder einem Fass oder direkt in der Gießmasse.

Im Gegensatz zum Silanisierungsverfahren, das eine siebentägige Lagerung des behandelten Füllstoffs für die Nachreaktion erfordert, schlägt die vorliegende Erfindung eine Technologie vor, die es erlaubt, den Füllstoff direkt nach der Behandlung zu verwenden. Ferner erfordert das erfindungsgemäße Verfahren keinen Erhitzungsprozess, wohingegen die Silanisierungsreaktion bei der Erhitzung auf mindestens 60 °C für mindestens 30 Minuten durchgeführt wird.

Hydrophobierte und aktivierte Füllstoffe der vorliegenden Erfindung haben eine unterschiedliche Grenzfläche mit der Matrix. Die, nahe der Füllstoffoberfläche lokalisierte Doppelbindung der Methacryloylgruppe kapselt die Füllstoffoberfläche während der Polymerisation ein und hält die Doppelbindung an den Seitenketten der Fettsäure für die Copolymerisation mit der Matrix bereit. Diese führen zum Aufbau der weniger gespannten Füller-Matrix-Grenzfläche. Das reduziert Spannungen lokal und in den Formteilen insgesamt.

Die Mengen an hydrophobierender und zu aktivierender, reaktiver Substanz auf natürlicher Basis sind abhängig von der spezifischen Füllstoffoberfläche und werden daher in g/m2 angegeben. Hierbei wird die Menge am eingesetzten Methacryloylmonomer so gewählt, dass ein Aufbau einer Monoschicht des Methacryloylmonomers über die Amingruppe mit der Hydroxylgruppe des Füllstoffs bevorzugt wird. Die Menge hängt daher von der Dichte der Hydroxylgruppen auf der Füllstoffoberfläche ab. Beispielsweise kann von 0,8 -OH-Gruppen pro nm² und 0,2 m²/g bei einem Quarzsand ausgegangen werden und hierüber die notwendige Menge an Methacryloylmonomer bestimmt werden.

Die Mischzeit in dem Mischaggregat, z.B. ein Röhnrad-Mischer, wird ebenfalls in Abhängigkeit von der Zusammensetzung der Füllstoffe variiert. Partikel mit einem größeren Durchmesser benötigen weniger Zeit, um eine gleichmäßige Verteilung der ölbasierten Monomere über die Füllstoffoberfläche zu erreichen. Bei feinen Partikeln, Quarz oder Fruchtkernmehl ist die Mischzeit länger.

Erfindungsgemäß wird ein anorganischer und/oder organischer Füllstoff bereitgestellt, der eine hydrophobierte und aktivierte Oberfläche aufweist. Die anorganischen Füllstoffe können ausgewählt werden aus SiO₂, Al₂O₃, TiO₂, ZrO₂, Fe₂O₃, ZnO, Cr₂O₅, Kohlenstoff, Metallen und Metalllegierungen, SiC, SiN, BN oder Mischungen davon.

Die organischen Füllstoffe sind gemahlene Fruchtkerne und/oder Fruchtschalen und können aus Olivenkernen, Pfirsichkernen, Aprikosenkernen, Kirschkernen, Mandelschalen, Arganschalen, Walnussschalen oder einer Mischung davon ausgewählt werden.

Die anorganischen und organischen Füllstoffe können in einer Kombination aus beiden Arten von Füllstoffen verwendet werden. Das Mischungsverhältnis kann beliebig gewählt werden.

Die hydrophobierte und aktivierte Oberfläche des anorganischen und/oder organischen Füllstoffs wird durch Immobilisierung mindestens eines biobasierten (meth)acrylierten Monomers gebildet, das eine aus biologischem Anbau stammende Fettsäuregruppe umfasst, die mit der (Meth)acrylgruppe an der Oberfläche des anorganischen und/oder organischen Füllstoffs verestert ist.

Die in Schritt (a) verwendeten anorganischen und organischen Füllstoffe können eine Korngröße von 1 µm bis 2000 µm aufweisen. Die vorliegende Erfindung basiert auf dem Konzept der Hydrophobierung und Aktivierung von anorganischen und/oder organischen Füllstoffen einschließlich einer Oberflächenbehandlung mit mindestens einer reaktiven Verbindung auf biologischer Basis.

Die hydrophobierte und aktivierte Oberfläche des anorganischen und/oder organischen Füllstoffs wird durch die Immobilisierung mindestens eines biobasierten (meth)acrylierten Monomers erhalten, das eine, aus biologischem Anbau stammende Fettsäuregruppe, enthält, die eine Gruppe umfasst, die mit der (Meth)acrylgruppe verestert ist. Das biobasierte (meth)acrylierte Monomer wird in dem Monomer gelöst, das in der Polymermatrix des Formteils enthalten ist. Die Konzentration des biobasierten (meth)acrylierten Monomers sollte 1 bis 20 Gew.-%, vorzugsweise 3 bis 17,5 Gew.-%, insbesondere 5 bis 15 Gew.-% betragen.

Dabei kann die in Schritt (b) verwendete hydrophobierende und zu aktivierende, reaktive Verbindung auf biologischer Basis ausgewählt sein aus dem Methacryloylmonomer auf Basisvon Ölen natürlichen Ursprungs der allgemeinen Formel: H2C=C(R1)C(O)-NH-CH2-CH2-C(O)-O-R2, wobei R1 im Falle von Acryl H und im Falle von Methacryl CH3 ist, wobei R2 ein Fettsäurerest aus den Ölen natürlichen Ursprungs ist, der in der Masse mit dem N-Hydroxyethyl(meth)acrylamid reagiert.

Weiterhin kann die in Schritt (b) verwendete hydrophobierende und zu aktivierende, biobasierte, reaktive Verbindung in dem mindestens einen, in der Polymermatrix des Formteils vorhandenen Monomer gelöst werden.

Als Lösungsmittel können monofunktionelle Monomere in Form eines Acrylatmonomers verwendet werden. Diese können ausgewählt werden aus Methylacrylat, Ethylacrylat, n-Butylacrylat, tert. Butylacrylat, Isobutylacrylat, Isodecylacrylat, Dihydroxycyclopentadienylacrylat, Ethyldiglycolacrylat, Heptadecylacrylat, 4-Hydroxybutylacrylat, 2-Hydroxyethylacrylat, Hydroxyethylcaprolactonacrylat, Polycaprolactonacrylat, Hydroxypropylacrylat, Laurylacrylat, Stearylacrylat, 2-(2-Ethoxy)ethylacrylat, Tetragydroxyfurfurylacrylat, 2-Phenoxyethylacrylat, ethoxyliertes 4-Phenylacrylat, Trimethylcyclohexylacrylat, Octyldecylacrylat, Tridecylacrylat, ethoxyliertes 4-Nonylphenylacrylat, Isobornylacrylat, cyclisches Trimethylolpropanformalacrylat, ethoxyliertes 4-Laurylacrylat, Polyesteracrylat, hyperverzweigtes Polyesteracrylat, Melaminacrylat, Siliconacrylat, Epoxyacrylat.

Darüber hinaus ist es möglich, ein monofunktionelles Monomer in Form eines Methacrylats zu verwenden. Dieses kann ausgewählt werden aus Methylmethacrylat, Ethylmethacrylat, n-Butylmethacrylat, Isobutylmethacrylat, tert-Butylmethacrylat, Behenylmethacrylat, Behenyl-polyethylenglykolmethacrylat, Cyclohexylmethacrylat, Isodecylmethacrylat, 2-Ethylhexylmethacrylat, Laurylmethacrylat, Stearylmethacrylat, Stearylpolyethylenglykolmethacrylat, Isotridecylmethacrylat, Ureidomethacrylat, Tetrahydrofurfurylmethacrylat, Phenoxyethylmethacrylat, Isobornylmethacrylat, Methoxypolyethylenglykolmethacrylat, Glycidylmethacrylat, Glycerinformalmethacrylat, Lauryltetradecylmethacrylat.

Als Lösungsmittel kann auch ein polyfunktionelles Monomer in Form eines polyfunktionellen Acrylats verwendet werden. Dieses kann ausgewählt werden aus 1,6-Hexandioldiacrylat, Polyethylenglykoldiacrylat, Polybutadiendiacrylat, Tetraethylenglykoldiacrylat, 3-Methyl-1,5-pentandioldiacrylat. Ethoxyliertes Bisphenol-A-Diacrylat, Dipropylenglykoldiacrylat, ethoxyliertes Hexandioldiacrylat, 1,10-Decandioldiacrylat, Esterdiacrylat, alkoxyliertes Diacrylat, Tricyclodecandimethanoldiacrylat, propoxyliertes Neopentylglykoldiacrylat, Pentaerythrit-tetraacryllat, Trimethylolpropantriacrylat, ethoxyliertes Trimethylolpropantriacrylat, Ditrimethylolpropantetraacrylat, Tris(2-hydroxyethyl)isocyanurattriacrylat, Dipentaerythritol-pentaacrylat, Pentaerythritoltriacrylat, propoxyliertes Glycerintriacrylat, aliphatisches Urethan-triacrylat, aliphatisches Urethandiacrylat, aromatisches Urethandiacrylat, aromatisches Urethantriacrylat, aromatisches Urethanhexaacrylat, Polyesterhexaacrylat, epoxidiertes Sojabohnenöldiacrylat.

Außerdem kann ein polyfunktionelles Biomonomer in Form eines biobasierten Methacrylats verwendet werden. Dieses kann ausgewählt werden aus Triethylenglykoldimethacrylat, Ethylenglykoldimethacrylat, Polyethylenglykoldimethacrylat, 1,4-Butandioldimethacrylat, Diethylenglykoldimethacrylat, 1,6-Hexandioldimethacrylat, 1,10-Decandioldimethacrylat, 1,3-Butylenglykoldimethacrylat, Tricyclodecandimethanoldimethacrylat, Trimethylolpropantrimeth-acrylat.

Es ist möglich, Fettsäuren in Form von Pflanzenölen zu verwenden. Dieses kann ausgewählt werden aus Kokosöl, Keimöl, Rapsöl, Baumwollsamenöl, Oliveöl, Palmöl, Erdnusöl, Distelöl, Sesamöl, Sojabonenöl, Sonnenblumenöl, Mandelöl, Bucheckernöl, Paranussöl, Cashewöl, Hazelnussöl, Macadamiaöl, Mongongoöl, Pecanöl, Pistazienöl, Walnusöl, Kurbiskernöl, Grapefruitkernöl, Lemonöl, Orangeöl, Bittermeloneöl, Calabashöl, Cucurbitaöl, Butternussamenöl, Egusisamenöl, Wassermelonesamenöl, Boragesamenöl, Johannisbeeresamenöl, Schwarzsamenöl, Acaiöl, Nachtkerzenöl, Leinöl, Amaranthöl, Aprikosenkernenöl, Apflesamenöl, Arganöl, Avocadoöl, Babassuöl, Behenöl, Salnusöl, Kapkastanienöl, Algorabaöl, Kakaobutter, Spitzklettenöl, Kohunöl, Koriandersamenöl, Datelnsamenöl, Dikaöl, Traubenkernöl, Hanföl, Kapoksamenöl, Kenafsamenöl, Lallemantiaöl, Marula Öl, Senföl, Ramtilkrautöl, Muskatnussbutter, Ochersamenöl, Perillasamenöl, Kakisamenöl, Pequi-ÖI, Pillinussöl, Granatapfeklernöl, Mohnöl, Placaxi-ÖI, Pflaumenkernöl, Quinoaöl, Reisöl, Sacha-Inchi-ÖI, Sapote Öl, Patawaöl, Shea Butter, Taramira ÖI, Teasamenöl, Erdmandelöl, Tobaccosamenöl, Tomatensamenöl, Weizenkaimöl, Rezinusöl, Leindotteröl, Rattichöl, Salicornia öl, Tung ÖI, Kopaiba Oleoresin, Jatropha ÖI, Jojobaöl, Nagkesaröl, Pongamiaöl, Dammaröl, Stilingia oil, Artischocke ÖI, Murumuru Butter, Balanos ÖI, Bladderpod ÖI, Macassarkernöl, Klettenwurzelöl, Buriti ÖI, Kukuinussöl, Karrotensamenöl, Kuphea ÖI, Mangoöl, Passionblumenöl, Hagebuttenkernöl, Gummi Samen ÖI, Sanddornöl, Tamanuöl, Tonkabonenöl.

Darüber hinaus ist es möglich, eine Fettsäure in Form eines ätherischen Öls zu verwenden. Dieses kann ausgewählt werden aus Oudöl, Ajowanöl, Angelikawurzelöl, Anisöl, Asafoetidaöl, Basilikumöl, Perubalsam, Lorbeeröl, Bergamotöl, Pfefferöl Schwarz, Buchuöl, Birchöl, Kampheröl, Calamodinöl, Kümmelöl, Kardamomöl, Zedernholzöl, Kamilienöl, Kalmusöl, Zimtöl, Zitronenöl, Zitrongrasöl, Salbeiöl, Nelkenöl, Kaffeeöl, Korianderöl, Costmaryöl, Kostuswurzelöl, Preiselbeerensamenöl, Kubebenöl, Kreuzkümmelöl, Zypressenöl, Curryblatt-öl, Davanaöl, Dillöl, Immortelleöl, Elemiöl, Eucalyptusöl, Fenchelsamenöl, Galangalöl, Galbanumöl, Knoblauchöl, Geraniumöl, Ingweröl, Hennaöl, Strohblumenöl, Meerrettichöl, Jasminöl, Wacholderbeerenöl, Lavendelöl, Melissenöl, Moringaöl, Mugwortöl, Myrrhenöl, Neemöl, Oreganoöl, Nardenöl, Petersillenöl, Patchouliöl, Perillaöl, Pfefferminzöl, Pinienkernöl, Rosmarinöl, Sandelholzöl, Sassafrasöl, Bohnenkrautöl, Schisandrabeerenöl, Minzöl, Thymianöl.

Außerdem kann eine Fettsäure in Form eines tierischen Fetts und / oder Öls verwendet werden. Dieses kann ausgewählt werden aus Fischöl, Bärengras, Hähnchenfett, Krokodilfett, Krokodilöl, Dorschleberöl, Emuöl, Schweinefett, Gänsefett, Entenfett, Haileberöl

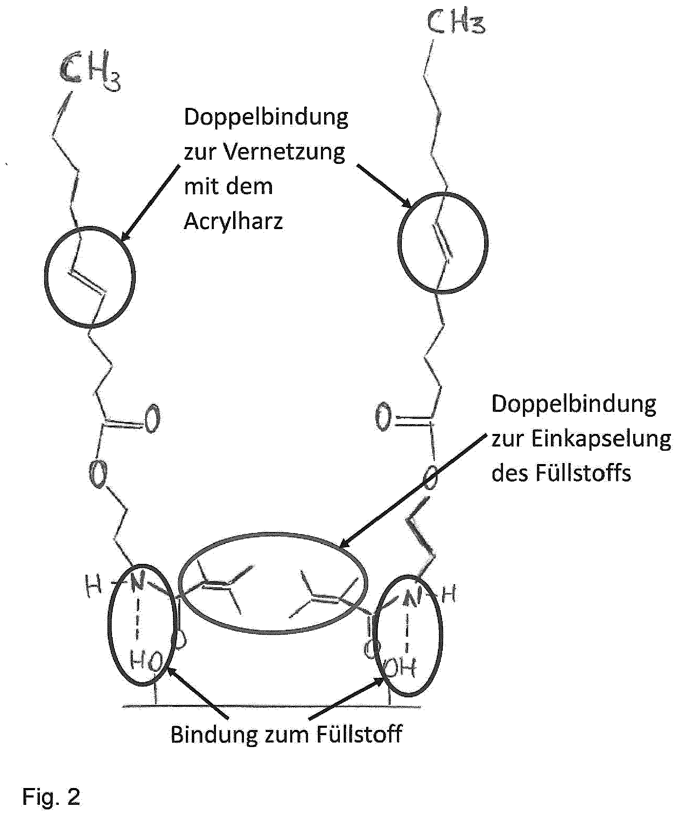

Erfindungsgemäß wird die so hergestellte Lösung aus dem Lösungsmittel und dem, mit einer Fettsäuregruppe funktionalisierten Acrylamid im rotierenden Pulvermischer auf der Oberfläche der festen Füllstoffe abgeschieden. Die (Meth)acrylgruppen in den reaktiven (Meth)acrylmonomeren verestern zu einer Polymermatrix, um auf der Oberfläche der anorganischen und/oder organischen Füllstoffpartikel immobilisiert zu werden (siehe Fig. 2). Dort ist schematisch der Prozess von Füllstoffverkapselung und Verbindung zum Polymerbinder dargestellt.

Die Oberfläche anorganischer und/oder organischer Füllstoffpartikel ist durch das Vorhandensein funktioneller Gruppen, wie z. B. einer Hydroxyl-Gruppe auf der Quarzoberfläche, funktional. Diese funktionellen Hydroxylgruppen werden als Immobilisierungszentren für die biologisch abgeleiteten Fettsäuremonomermoleküle der Erfindung verwendet. Die Fettsäure-Acrylamid-Moleküle chemosorbieren auf der Füllstoffoberfläche und bilden eine reaktive (Meth)acrylatschicht mit organischen Ketten, die von der Oberfläche nach außen gerichtet sind. Der Wassertropfen, der auf die mit dem Acrylmonomer z.B. auf Olivenölbasis modifizierte Quarzoberfläche der Quarzpartikel aufgebracht wird, verbleibt mehr als 240 Sekunden lang auf der Oberfläche der so zu einem Pressling verdichteten Füllstoffpartikel. Derselbe Effekt wird bei Verwendung anderer ölbasierter (Meth)acrylate erzielt. Die Doppelbindung, die mit dem (Meth)acrylamid verbunden ist, ist nahe der Füllstoffoberfläche lokalisiert. Eine solche Morphologie ermöglicht den Aufbau einer gleichmäßigen organischen Schicht auf der Füllstoffoberfläche während der Polymerisation. Andererseits nimmt die Doppelbindung der ungesättigten Fettsäuren, die weit von der Füllstoffoberfläche entfernt ist, am Copolymerisationsprozess mit den Matrixmonomeren teil. Diese Dimensionsspezifität in der Bindung zwischen Matrix und Füllstoff erhöht die Schlagzähigkeit und die Temperaturwechselbeständigkeit, verringert die Auswirkungen des Vernetzungsgrades und führt damit zur Verringerung der Zerbrechlichkeit des Formkörpers.

Neben dem Verfahren betrifft die Erfindung ferner einen hydrophoben und reaktiven anorganischen und/oder organischen Füllstoff, hergestellt nach dem erfindungsgemäßen Verfahren.

Anorganische und organische Füllstoffe gemäß der Erfindung weisen eine bevorzugt mit mindestens einem biobasierten (Meth)acrylatmonomer behandelte Oberfläche auf, die eine biologisch abgeleitete Fettsäuregruppe mit einer an die (Meth)acrylatgruppe veresterten Gruppe enthält. Verbundwerkstoffe, die diese offengelegten, anorganischen und/oder organischen Füllstoffe mit einer mit mindestens einem biobasierten (Meth)acrylatmonomer behandelten Oberfläche enthalten, können die Reduzierung der Spannungen während der Aushärtung des Formteils zeigen. Das hier offengelegte gehärtete Formteil kann reduzierte Spannungen aufweisen, wodurch die mechanischen Eigenschaften des polymerisierten Verbundstoffs zufriedenstellend verbessert werden.

Weiterhin betrifft die Erfindung die Verwendung eines solchen Füllstoffs als Zuschlag zu einer polymerbasierten Gießmasse. Eine solche Gießmasse dient der Herstellung von gegossenen und ausgehärteten Formteilen, z.B. in Form von Küchenspülen, Duschtassen etc.

Schließlich betrifft die Erfindung ein ausgehärtetes Formteil z.B. in Form eines Küchen- oder Sanitärartikels, z.B. einer Küchenspüle oder Duschtasse, hergestellt unter Verwendung einer solchen Gießmasse.

Weitere Vorteile und Einzelheiten der Erfindung ergeben sich aus den im Folgenden beschriebenen Ausführungsbeispielen sowie anhand der Zeichnungen. Dabei zeigen:
- Fig. 1: zeigt die vergleichenden Spektren des unbehandelten Quarzmehls (unten) und des hydrophobierten und aktivierten Quarzmehls.
- Fig. 2: eine schematische Darstellung des Vorgangs der Füllstoffverkapselung und Verbindung zum Polymerbinder

Im Folgenden wird ein Versuchsbeispiel vorgestellt, um die anorganischen und/oder organischen Füllstoffe einschließlich der hydrophobierten und aktivierten Oberfläche der Erfindung, die Gießmasse der Erfindung und die Formgebung der Erfindung im Detail zu illustrieren.

### Beispiel

### Hydrophobierung und Aktivierung der anorganischen und/oder organischen Füllstoffe

Verwendete Komponenten:
a) Anorganische und / oder organische Füllstoffe:
   Quarzsand (Partikelgröße 0,06 bis 0,3 mm, Hersteller: Dorfner GmbH), Quarzmehl (1 bis 50 µm, Dorfner GmbH), Cristobalitmehl (0,1 bis 10 µm, Quartzwerke GmbH) Olivenkernmehl (1,0 - 100 µm, BioPowder Ltd) Olivenkernpartikel (600 - 800 µm, BioPowder Ltd), Pfirsichkernpartikel (300 - 600 µm, BioPowder Ltd)
b) Biobasierte Monomere:
   Isobornylmethacrylat, (IBOMA, Evonik Performance Materials GmbH), Polyethylenglykol 200 Dimethacrylat (PEG-200-DMA, Fa. Arkema)
c) Methacryloylmonomere auf Pflanzenölbasis:
   Monomer auf Olivenölbasis (OBM, North Dakota State University), Monomer auf Sojabohnenölbasis (SBM, North Dakota State University)

Die Zusammensetzungen zur Herstellung von Hydrophobierungs- und Aktivierungsmitteln werden durch Auflösen von auf Pflanzenöl basierenden Methacryloylmonomeren (OBM und/oder SBM, North Dakota State University) in den biobasierten Monomeren (IBOMA, (Evonik Performance Materials GmbH) und/oder PEG-200-DMA (Arkema) hergestellt. Das Reaktionsgemisch wurde bei 35 °C 40 Minuten lang beschallt (Bandelin Super RK 1028 H Ultraschallbad), bis eine klare gelbliche Lösung erhalten wurde. Zum Vergleich der Hydrophobierungs- und Aktivierungsmittel wurden die Zusammensetzungen hergestellt, die in Tabelle 1 zusammengefasst sind. Die Angaben sind in Gewichts-Prozent angegeben.

**TABELLE 1**

| | Muster 1 | Muster 2 | Muster 3 | Muster 4 | Muster 5 |
|---|---|---|---|---|---|
| IBOMA | 90 | 85 | 60 | 20 | |
| PEG-200-DMA | | | 31 | 65 | 90 |
| OBM | 10 | 2 | | 15 | 5 |
| SBM | | 13 | 9 | | 5 |

Alle Muster aus Tabelle 1 wurden als Hydrophobierungs- und Aktivierungsmittel für die Behandlung der anorganischen und/oder organischen Füllstoffe in den verschiedenen Verhältnissen entsprechend der spezifischen Oberfläche der Füllstoffpartikel verwendet (0,221 m²/g für Quarzsand; 1,5 m²/g für Quarzmehl; 3,5 m²/g für Cristobalitmehl; 2,6 m²/g - Olivenkernmehl; 0,32 und 0,27 m²/g für Oliven- bzw. Pfirsichkernpartikel). Die Angaben beziehen sich auf die spezifische Oberfläche pro Gramm Füllstoff.

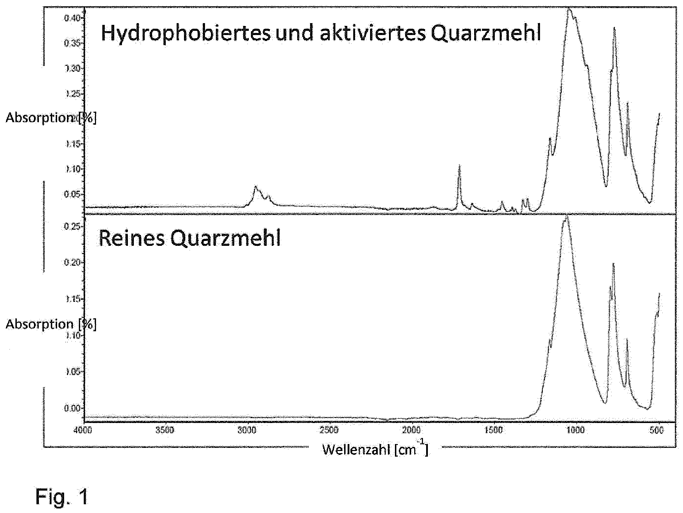

Die klare Lösung der Methacryloylmonomere auf Pflanzenölbasis der Mustern 1 - 5 wurde zur Hydrophobierung und Aktivierung der anorganischen und/oder organischen Füllstoffoberfläche verwendet. Die entsprechende Menge der Lösung wurde den Füllstoffen, wie Quarzsand (Teilchengröße 0,06 bis 0,3 mm, Dorfner GmbH), Quarzmehl (1 bis 50 µm, Dorfner GmbH), Cristobalitmehl (0,1 bis 10 µm, Quartzwerke GmbH), Olivenkernmehl (1,0 - 100 µm, BioPowder Ltd), Olivenkernpartikel (600 - 800 µm, BioPowder Ltd), Pfirsichkernpartikel (300 - 600 µm, BioPowder Ltd), zugegeben und in Mischzylinder gegeben. Die Zylinder wurde geschlossen und auf Rotationswalzen gestellt, um die Füllstoffpartikel mit dem Hydrophobierungs- und Aktivierungsmittel gleichmäßig zu benetzen. Die auf diese Weise hergestellten Gemische wurden 2 Stunden lang bei einer Rotationsgeschwindigkeit von 30 U/min gerührt. Anschließend wurden die so hydrophobierten Füllstoffe aus dem Behälter entnommen und zur weiteren Verwendung für die Herstellung von Gießmassen überführt. Figur 1 zeigt das IR-Spektrum des Quarzmehls (wie erhaltenes Quarzmehl, unten) und das IR-Spektrum des mit Methacryloylmonomer auf Olivenölbasis behandelten Quarzmehls. Ein intensiver Peak bei ca. 1650 cm-1 bestätigt eindeutig das Vorhandensein reaktiver Doppelbindungen, die mit den Matrixmonomeren co-polymerisieren können.

Tabelle 2 fasst die Füllstoffzusammensetzungen zusammen, die mit den ölbasierten Monomeren der Tabelle 1 hydrophobiert und aktiviert wurden. Die Angaben sind in Gewichts-Prozent angegeben.

**TABELLE 2**

| | Muster 1 | Muster 2 | Muster 3 | Muster 4 | Muster 5 |
|---|---|---|---|---|---|
| Quarzsand 0,06 bis 0,3 mm | 90 | 85 | 60 | 40 | 50 |
| Quarzmehl 1 bis 50 µm | | | 31 | | 20 |
| Cristobalitmehl 0,1 to 10 µm | 10 | 2 | | 15 | |
| Olivenkernmehl 1,0 - 100 µm | | 13 | 9 | | 5 |
| Olivenkerngranulat 600 - 800 µm | | | | 45 | |
| Pfirsichkerngranulat 300 - 600 µm | | | | | 25 |

Für die Herstellung der Gießmassen und die anschließende Aushärtung in der jeweiligen Form wurden diese Füllstoffmischungen (Muster 1 bis 5) verwendet.

Die typische Formulierung kann wie folgt beschrieben werden:
23,7 kg recyceltes PMMA (XP-95, KFG, Deutschland) wurden in der Mischung aus 56,3 kg recyceltem Methylmethacrylat (r-MMA, Monomeros des Valles, Spanien), 15 kg Isobornylmethacrylat, Visiomer Terra IBOMA (Evonik Performance Materials, Deutschland) und 5 kg biobasiertem Ethylmethacrylat (BCH-Bruehl, Deutschland) bis zur Erzielung einer klaren Lösung aufgelöst. 0,1 kg biobasierte Stearinsäure (Musim Mas, Singapur) wurde der PMMA-Lösung in Monomeren zugesetzt. Nachdem sich die Stearinsäure vollständig aufgelöst hatte, wurden 4,0 kg Sarbio 6201, Polyethylenglykol(200)-dimethacrylat (Arkema, Frankreich) zur PMMA-Lösung hinzugefügt. 210 kg des Füllstoffsystems aus den Muster 1 bis 5 wurden in dieser Mischung dispergiert.

Diese Lösung wurde für die Herstellung der Muster 1 - 5 verwendet, indem die entsprechenden hydrophobierten Füllstoffmischungen dispergiert wurden, gefolgt von der Zugabe des Initiatorsystems (2 Gew.-%, berechnet aus der Monomermenge) aus der Mischung von Perkadox 16 und Laurox S (Nouryon, Niederlande) im Verhältnis 1:2.

Nach Zugabe des Initiatorsystems und 15-minütiger Entgasung wurde die Gießmasse in die geschlossene Form gespritzt und zum Aushärten 30 Minuten lang auf 100 °C erhitzt und anschließend abgekühlt, wonach die hergestellten Formteile den Formen entnommen wurden.

Parallel wurden Vergleichsformteile unter Verwendung einer jeweils identischen Gießmasse, in der jedoch anstelle der erfindungsgemäß behandelten Füllstoffe gleiche, jedoch unbehandelte Füllstoffe in identischer Konzentration enthalten waren, hergestellt, um die Eigenschaften von Formteilen mit erfindungsgemäß behandelten Füllstoffen mit den Eigenschaften gleicher Formteile mit unbehandelten Füllstoffen vergleichen zu können.

Die mechanischen und thermischen Eigenschaften der Formteile der Muster 1 - 5 und der Vergleichsformteile (1a - 5a), die unter Verwendung der unbehandelten Füllstoffe in gleichen Konzentrationen hergestellt wurden, wurden mit denen der erfindungsgemäßen Muster verglichen.

**TABELLE 3**

| | Formteil 1/1a | Formteil 2/2a | Formteil 3/3a | Formteil 4/4a | Formteil 5/5a |
|---|---|---|---|---|---|
| Schlagzähigkeit, mJ/mm² | 3,7/3,3 | 3,3/3,0 | 3,4/3,2 | 3,4/3,2 | 3,5/3,2 |
| Kratztest | +/+ | +/+ | +/+ | +/+ | +/+ |
| Taber Abrieb, mg | 22/20 | 20/20 | 23/21 | 21/19 | 20/19 |
| Beständigkeit gegen trockene Wärme | +/+ | +/+ | +/+ | +/+ | +/+ |
| Temperaturwechselbeständigkeit | +/+ | +/+ | +/+ | +/+ | +/+ |

Für die Schlagzähigkeitsmessungen wurden 12 Proben mit einer Größe von 80 x 6 mm aus dem Formteil geschnitten. Die Messungen wurden mit einem Pendelschlaggerät ZwickRoell HIT P Gerät durchgeführt.

Für die Messung der Kratzfestigkeit wurde eine Probe (100 x 100 mm) ausgeschnitten, gemäß DIN EN 13310 getestet (Erichsen 213 Kratzgerät) und die Topografie vor und nach dem Kratzen gemessen (Mitutoyo Surftest SJ 500 P Rauheitsmessgerät)

Für den Taber-Abriebtest wurde eine Probe (100 x100 mm) geschnitten und ein Abriebtest mit einem Elcometer 1720 durchgeführt.

Die Beständigkeit gegen trockene Wärme basiert auf der Prüfmethode DIN EN 13310, bei der das Prüfstück mit einer Temperatur von 180 °C 20 Minuten lang in die Mitte des zu prüfenden Formteils gelegt wird, ohne sichtbare Veränderungen an der Struktur der Spüle zu hinterlassen.

Das Prüfverfahren der Temperaturwechselbeständigkeit basiert auf dem Prüfverfahren DIN 13310, bei dem das Prüfformteil (Küchenspüle) 1000 Zyklen lang mit Kalt-Heißwasser behandelt wird. Dabei fließt 90 Sekunden lang heißes Wasser (T=90°C) in die Spüle, gefolgt von einer Ruhephase von 30 Sekunden, in der wiederum 90 Sekunden lang kaltes Wasser (T=15°C) fließt. Der Zyklus wird durch eine Entspannung von 30 Sekunden beendet.

Wie die Messergebnisse zeigen, weisen nahezu alle erfindungsgemäßen Formteile verbesserte Eigenschaften gegenüber den Vergleichsformteilen auf.

So hat sich die Schlagzähigkeit deutlich verbessert, zum Teil um 10% verglichen mit dem Vergleichsformteil.

Gleiches gilt auch für den Taber-Abrieb.

Auch erfüllten alle erfindungsgemäßen Formteile die testgemäßen Anforderungen hinsichtlich der Kratzfestigkeit, der Beständigkeit gegen trockene Wärme und der Temperaturwechselbeständigkeit.

Figur 1 zeigt die IR-Spektren des Quarzmehls vor und nach der Behandlung mit Methacryloylmonomer auf Olivenölbasis. Im Spektrum des behandelten Füllstoffs ist deutlich die Doppelbindung des Monomers zu erkennen, die mit den Matrixmonomeren polymerisiert und die weniger belastete Grenzfläche zwischen Füllstoff und Matrix sowie das Formteil insgesamt bildet. Der intensive Peak bei 1650 cm-1 bestätigt eindeutig das Vorhandensein einer reaktiven Doppelbindung des auf Naturöl basierenden Monomers, das durch Pfropfen auf die Matrix copolymerisieren kann.

Figur 2 zeigt das Schema der Quarzsandoberfläche, die mit dem Methacryloylpolymer auf Olivenölbasis behandelt wurde, wobei die Doppelbindungen des Methacryloylteils, die die Verkapselungsschicht auf der Sandoberfläche bilden, und die Doppelbindungen der labilen Ketten, die mit den Matrixmonomeren copolymerisieren, zu sehen sind. Die lange CH2-CH2-Fettsäurekette ermöglicht eine flexible Reaktion auf mechanische und thermische Beanspruchungen. Die Aminogruppe des Methacryloylteils der Moleküle schafft eine starke Bindung mit der Füllstoffoberfläche.

## Patentansprüche

1. Verfahren zur Herstellung von hydrophoben und reaktiven anorganischen und/oder organischen Füllstoffen, umfassend die Schritte: (a) Bereitstellen eines Füllstoffs mit einer bestimmten Oberfläche, (b) Mischen des Füllstoffs mit der Lösung von mindestens einer hydrophobierenden und aktivierenden reaktiven Verbindung auf biologischer Basis in einem Mischaggregat in einer Menge von 0,15 × 10⁻² bis 5,0 × 10⁻² g pro m² Füllstoffoberfläche bei einer Geschwindigkeit von 20 U/min bis 200 U/min für 12 Minuten bis 120 Minuten, (c) Evakuieren des hydrophobierten und aktivierten anorganischen und/oder organischen Füllstoffs in einem Lagerbeutel, einer Box oder einem Fass oder direkt in der Gießmasse.

2. Verfahren nach Anspruch 1, **dadurch gekennzeichnet, dass** in Schritt (a) der anorganische Füllstoff ausgewählt wird aus SiO₂, Al2O₃, TiO₂, ZrO₂, Fe₂O₃, ZnO, Cr₂O₅, Kohlenstoff, Metallen und Metalllegierungen, SiC, SiN, BN oder einer Mischung davon.

3. Verfahren nach Anspruch 1, **dadurch gekennzeichnet, dass** in Schritt (a) der organische Füllstoff aus gemahlenen Fruchtkernen und/oder Fruchtschalen ausgewählt wird und aus Olivenkernen, Pfirsichkernen, Aprikosenkernen, Kirschkernen, Mandelschalen, Arganschalen, Walnussschalen oder einer Mischung davon ausgewählt werden kann.

4. Verfahren nach Anspruch 2 und 3, **dadurch gekennzeichnet, dass** in Schritt (a) die anorganischen und organischen Füllstoffe in einer Kombination beider Arten von Füllstoffen in einem beliebigen Mischungsverhältnis verwendet werden können.

5. Verfahren nach einem der vorangehenden Ansprüche, **dadurch gekennzeichnet, dass** in Schritt (a) die anorganischen und organischen Füllstoffe eine Korngröße von 1 µm bis 2000 µm aufweisen können.

6. Verfahren nach einem der vorangehenden Ansprüche, **dadurch gekennzeichnet, dass** in Schritt (b) die hydrophobierende und aktivierende reaktive Verbindung auf biologischer Basis ausgewählt ist aus dem Methacryloylmonomer auf Pflanzenölbasis der allgemeinen Formel: H₂C=C(R₁)C(O)-NH-CH₂-CH₂-C(O)-O-R₂, wobei R₁ im Falle von Acryl H und im Falle von Methacryl CH₃ ist, wobei R₂ ein Fettsäurerest aus den Ölen auf Pflanzenölbasis ist, der in Masse mit dem N-Hydroxyethyl(meth)acrylamid reagiert.

7. Verfahren nach einem der vorangehenden Ansprüche, **dadurch gekennzeichnet, dass** in Schritt (b) die hydrophobierende und aktivierende biobasierte reaktive Verbindung in dem mindestens einen in der Polymermatrix des Formteils vorhandenen Monomer gelöst wird.

8. Verfahren nach einem der vorangehenden Ansprüche, **dadurch gekennzeichnet, dass** das in Schritt (b) als Lösungsmittel für die hydrophobierenden und aktivierenden reaktiven Verbindungen verwendete Monomer ausgewählt wird aus monofunktionellen Acrylmonomeren wie Methylacrylat, Ethylacrylat, n-Butylacrylat, tert. Butylacrylat, Isobutylacrylat, Isodecylacrylat, Dihydroxycyclopentadienylacrylat, Ethyldiglycolacrylat, Heptadecylacrylat, 4-Hydroxybutylacrylat, 2-Hydroxyethylacrylat, Hydroxyethylcaprolactonacrylat, Polycaprolactonacrylat, Hydroxypropylacrylat, Laurylacrylat, Stearylacrylat, 2-(2-Ethoxy)etha-lacrylat, Tetragydroxyfurfurylacrylat, 2-Phenoxyethylacrylat, ethoxyliertes 4-Phenylacrylat, Trimethyl-cyclohexylacrylat, Octyldecylacrylat, Tridecylacrylat, ethoxyliertes 4-Nonylphenylacrylat, Isobornylacrylat, cyclisches Trimethylolpropanformalacrylat, ethoxyliertes 4-Laurylacrylat, Polyesteracrylat, hyperverzweigtes Polyesteracrylat, Melaminacrylat, Silikonacrylat, Epoxyacrylat.

9. Verfahren nach einem der Ansprüche 1 bis 7, **dadurch gekennzeichnet, dass** das in Schritt (b) als Lösungsmittel für die hydrophobierenden und aktivierenden biobasierten reaktiven Verbindungen verwendete Monomer ausgewählt wird aus monofunktionellen Methacrylmonomeren wie Methylmethacrylat, Ethylmethacrylat, n-Butylmethacrylat, Isobutylmethacrylat, tert-Butylmethacrylat, Behenylmethacrylat, Behenylpolyethylen-glykolmethacrylat, Cyclohexylmethacrylat, Isodecylmethacrylat, 2-Ethylhexylmethacrylat, Laurylmethacrylat, Stearylmethacrylat, Stearylpolyethylenglykolmethacrylat, Isotridecylmethacrylat, Ureidomethacrylat, Tetrahydrofurfurylmethacrylat, Phenoxyethylmethacrylat, Isobornylmethacrylat, Methoxypolyethylenglykolmethacrylat, Glycidylmethacrylat, Glycerinformalmethacrylat, Lauryltetradecylmethacrylat.

10. Verfahren nach einem der Ansprüche 1 bis 7, **dadurch gekennzeichnet, dass** das in Schritt (b) als Lösungsmittel für die hydrophobierenden und aktivierenden biobasierten reaktiven Verbindungen verwendete Monomer ausgewählt wird aus polyfunktionellen Acrylmonomeren wie 1, 6-Hexandioldiacrylat, Polyethylenglykoldiacrylat, Polybuta-diendiacrylat, Tetraethylenglykoldiacrylat, 3-Methyl-1,5-pentandioldiacrylat, ethoxyliertes Bisphenol-A-Diacrylat, Dipropylenglykoldiacrylat, ethoxyliertes Hexandioldiacrylat, 1,10-Decandioldiacrylat, Esterdiacrylat, alkoxyliertes Diacrylat, Tricyclodecandimethanoldiacrylat, propoxyliertes Neopentylglycoldiacrylat, Pentaerythrittetraacrylat, Trimethylolpropantriacrylat, ethoxyliertes Trimethylolpropantriacrylat, Ditrimethylolpropantetraacrylat, Tris(2-hydroxyethyl)isocyanurat-triacrylat, Dipentaerythritpentaacrylat, Pentaerythrittriacrylat, propoxyliertes Glycerintriacrylat, aliphatisches Urethantriacrylat, aliphatisches Urethandiacrylat, aromatisches Urethandiacrylat, aromatisches Urethantriacrylat, aromatisches Urethanhexaacrylat, Polyesterhexaacrylat, epoxidiertes Sojabohnenöldiacrylat.

11. Verfahren nach einem der Ansprüche 1 bis 7, **dadurch gekennzeichnet, dass** das in Schritt (b) als Lösungsmittel für die hydrophobierenden und aktivierenden biobasierten reaktiven Verbindungen verwendete Monomer ausgewählt wird aus polyfunktionellen Methacrylmonomeren wie Triethylenglykoldimethacrylat, Ethylenglycol-dimethacrylat, Polyethylenglycoldimethacrylat, 1,4-Butandioldimethacrylat, Diethylenglycol-dimethacrylat, 1,6-Hexandioldimethacrylat, 1,10-Decandioldimethacrylat, 1,3-Butylen-glycoldimethacrylat, Tricyclodecandimethanoldimethacrylat, Trimethylolpropantrimethacrylat.

12. Verfahren nach einem der vorangehenden Ansprüche und Anspruch 6, **dadurch gekennzeichnet, dass** das verwendete Öl ein Pflanzenöl ist.

13. Verfahren nach Anspruch 12, **dadurch gekennzeichnet, dass** das Pflanzenöl gewählt wird aus Kokosöl, Keimöl, Rapsöl, Baumwollsamenöl, Oliveöl, Palmöl, Erdnusöl, Distelöl, Sesamöl, Sojabonenöl, Sonnenblumenöl, Mandelöl, Bucheckernöl, Paranussöl, Cashewöl, Hazelnussöl, Macadamiaöl, Mongongoöl, Pecanöl, Pistazienöl, Walnusöl, Kurbiskernöl, Grapefruitkernöl, Lemonöl, Orangeöl, Bittermeloneöl, Calabashöl, Cucurbitaöl, Butternussamenöl, Egusisamenöl, Wassermelonesamenöl, Boragesamenöl, Johannisbeeresamenöl, Schwarzsamenöl, Acaiöl, Nachtkerzenöl, Leinöl, Amaranthöl, Aprikosenkernenöl, Apflesamenöl, Arganöl, Avocadoöl, Babassuöl, Behenöl, Salnusöl, Kapkastanienöl, Algorabaöl, Kakaobutter, Spitzklettenöl, Kohunöl, Koriandersamenöl, Datelnsamenöl, Dikaöl, Traubenkernöl, Hanföl, Kapoksamenöl, Kenafsamenöl, Lallemantiaöl, Marulaöl, Senföl, Ramtilkrautöl, Muskatnussbutter, Ochersamenöl, Perillasamenöl, Kakisamenöl, Pequiöl, Pillinussöl, Granatapfeklernöl, Mohnöl, Placaxiöl, Pflaumenkernöl, Quinoaöl, Reisöl, Sacha-Inchi-Öl, Sapoteöl, Patawaöl, Shea Butter, Taramiraöl, Teasamenöl, Erdmandelöl, Tobaccosamenöl, Tomatensamenöl, Weizenkaimöl, Rezinusöl, Leindotteröl, Rattichöl, Salicorniaöl, Tungöl, Kopaiba Oleoresin, Jatrophaöl, Jojobaöl, Nagkesaröl, Pongamiaöl, Dammaröl, Stilingia oil, Artischockenöl, Murumuru Butter, Balanosöl, Bladderpod Öl, Macassarkernöl, Klettenwurzelöl, Buritiöl, Kukuinussöl, Karrotensamenöl, Kupheaöl, Mangoöl, Passionblumenöl, Hagebuttenkernöl, Gummisamenöl, Sanddornöl, Tamanuöl, Tonkabonenöl.

14. Verfahren nach einem der vorangehenden Ansprüche und Anspruch 6, **dadurch gekennzeichnet, dass** das verwendete Öl ein ätherisches Öl ist.

15. Verfahren nach Anspruch 14, **dadurch gekennzeichnet, dass** das ätherische Öl gewählt wird aus Oudöl, Ajowanöl, Angelikawurzelöl, Anisöl, Asafoetidaöl, Basilikumöl, Perubalsam, Lorbeeröl, Bergamotöl, Pfefferöl Schwarz, Buchuöl, Birchöl, Kampheröl, Calamodin ö, Kümmelöl, Kardamomöl, Zedernholzöl, Kamilienöl, Kalmusöl, Zimtöl, Zitronenöl, Zitrongrasöl, Salbeiöl, Nelkenöl, Kaffeeöl, Korianderöl, Costmaryöl, Kostuswurzelöl, Preiselbeerensamenöl, Kubebenöl, Kreuzkümmelöl, Zypressenöl, Curryblattöl, Davanaöl, Dillöl, Immortelleöl, Elemiöl, Eucalyptusöl, Fenhelsamenöl, Galangalöl, Galbanumöl, Knoblauchöl, Geraniumöl, Ingweröl, Hennaöl, Strohblumeöl, Meerrettichöl, Jasminöl, Wacholderbeerenöl, Lavendelöl, Melissenöl, Moringaöl, Mugwortöl, Myrrhenöl, Neemöl, Oreganoöl, Nardenöl, Petersillenöl, Patchouliöl, Perillaöl, Pfefferminzöl, Pinienkernöl, Rosmarinöl, Sandelholzöl, Sassafrasöl, Bohnenkrautöl, Schisandrabeerenöl, Minzöl, Thymianöl.

16. Verfahren nach einem der vorangehenden Ansprüche und Anspruch 6, **dadurch gekennzeichnet, dass** das verwendete Öl ausgewählt wird aus tierischem Fett und / oder Öls verwendet werden. Dieses kann ausgewählt werden aus Fischöl, Bärengras, Hänchenfett, Krokodilfett, Krokodilöl, Dorschleberöl, Emuöl, Schweinefett, Gänsefett, Entenfett, Haileberöl.

17. Verfahren nach einem der vorangehenden Ansprüche, **dadurch gekennzeichnet, dass** in Schritt (b) die Konzentration der hydrophobierenden und aktivierenden biobasierten reaktiven Verbindung in dem Monomer, das in der Polymermatrix des Formteils besteht und als Lösungsmittel verwendet wird, von 1 bis 20 Gew.-%, vorzugsweise von 3 bis 17,5 Gew.-%, insbesondere von 5 bis 15 Gew.-% betragen sollte.

18. Hydrophober und reaktiver anorganischer oder organischer Füllstoff, hergestellt nach dem Verfahren gemäß mindestens einem der Ansprüche 1 bis 17.

19. Verwendung eines Füllstoffs nach Anspruch 18 als Zuschlag zu einer polymerbasierten Gießmasse zur Herstellung eines Komposit-Formteils oder als Teil eines Zahnfüllungs-Verbundsystems.

20. Formteil, hergestellt aus einer Gießmasse nach Anspruch 19.

21. Formteil nach Anspruch 20, **dadurch gekennzeichnet, dass** es eine Küchenspüle, ein Sanitärgegenstand in Form eines Waschbeckens, einer Duschwanne, einer Badewanne, eines WCs oder eines Bidets ist.
